# EUROPÄISCHE PATENTANMELDUNG

(11) **EP 3 301 092 A2**
(43) Veröffentlichungstag der Anmeldung: **04.04.2018**
(21) Anmeldenummer: 18153575.8
(22) Anmeldetag: 26.01.2018
(51) Int. Cl.: C07D 317/72, C07D 491/10, C07D 498/10, A01N 43/32

(54) **VERFAHREN ZUR HERSTELLUNG VON SPIROKETAL-SUBSTITUIERTEN PHENYLACETYLAMINOSÄUREESTERN UND SPIROKETAL-SUBSTITUIERTEN CYCLISCHEN KETOENOLEN**

(71) Anmelder: Bayer CropScience Aktiengesellschaft, 40789 Monheim am Rhein (DE)
(72) Erfinder: Die Erfindernennung liegt noch nicht vor
(74) Vertreter: BIP Patents

(57) **Zusammenfassung**

Die vorliegende Erfindung betrifft ein neues Verfahren zur Herstellung von spiroketal-substituierten Phenylacetylaminosäureestern und spiroketal-substituierten cyclischen Ketoenolen sowie neue Intermediate bzw. Ausgangsverbindungen, welche in dem erfindungsgemäßen Verfahren durchlaufen bzw. verwendet werden.

## Beschreibung

Die vorliegende Erfindung betrifft ein neues Verfahren zur Herstellung von spiroketal-substituierten Phenylacetylaminosäureestern und spiroketal-substituierten cyclischen Ketoenolen sowie neue Intermediate bzw. Ausgangsverbindungen, welche in dem erfindungsgemäßen Verfahren durchlaufen bzw. verwendet werden. Spiroketal-substituierte cyclische Phenylacetylaminosäureester sind wichtige Zwischenprodukte zur Synthese von insektiziden, akariziden und herbiziden Wirkstoffen.

Es ist bereits bekannt geworden, dass bestimmte spiroketal-substituierte cyclische Ketoenole insektizide, akarizide oder herbizide Wirksamkeit zeigen (WO 99/16748, WO 06/089633). Eine bekannt gewordene Synthese (Verfahren (A)) solcher spiroketal-substituierter cyclischer Ketoenole geht von entsprechend spiroketal-substituierten Cyclohexanonen der allgemeinen Formel (I) aus, die in einer Bucherer-Bergs-Reaktion zu den spiroketal-substituierten Hydantoinen der allgemeinen Formel (II) umgesetzt werden. Alkalische Verseifung dieser Hydantoine ergibt die spiroketal-substituierten Aminosäuren der allgemeinen Formel (III). Diese Aminosäuren werden dann nach bekannten Methoden der organischen Chemie (beispielsweise durch Umsetzung mit einem Alkohol R⁷-OH und Thionylchlorid) zu den spiroketal-substituierten Aminosäureestern der allgemeinen Formel (IV; R⁷ gleich C₁-C₆-Alkyl) verestert. Diese Aminosäureester werden dann mit Phenylessigsäurechloriden der allgemeinen Formel (VII) am Stickstoff zu den Verbindungen der allgemeinen Formel (VIII) acyliert. Die Verbindungen der allgemeinen Formel (VIII) werden dann in einer Dieckmann-Reaktion durch Einwirkung einer starken Base wie Kalium-tertiärbutylat oder Natriummethanolat zu den spiroketal-substituierten cyclischen Ketoenolen der allgemeinen Formel (XI) cyclisiert. Dieses Verfahren (A) ist in Schema 1 abgebildet. Ein erheblicher Nachteil dieses Verfahrens (A) besteht darin, dass bei der Veresterung der Aminosäuren der allgemeinen Formel (III) unter sauren Bedingungen fast immer auch eine zumindest teilweise Umketalisierung des cyclischen Ketals zu einem acyclischen Ketal (geminale Bis(alkoxy)-Verbindung) der allgemeinen Formel (V) stattfindet. Außerdem zeigt sich in der Praxis, dass auch unter prinzipiell wasserfreien Bedingungen, wenn auch nur in geringen Anteilen, zusätzlich Ketone der allgemeinen Formel (VI) gebildet werden können. Man erhält dann also den Ester als ein Gemisch aus zumindest zwei Produkten der allgemeinen Formeln (IV) und (V), das auch in den folgenden Stufen des Verfahrens (A) zu entsprechenden Produktgemischen führt. Zusätzlich führt die leichte Hydrolysierbarkeit der acyclischen Ketale dazu, dass auch N-acylierte Ketone der allgemeinen Formel (X) entstehen (identisch mit den Produkten der N-Acylierung der Ketoverbindungen der allgemeinen Formel (VI). So erhält man also nach der N-Acylierung mit einem Phenylessigsäurechlorid der allgemeinen Formel (VII) die Amide der allgemeinen Formeln (VIII), (IX) und (X). Die Dieckmann-Cyclisierung führt dann zu einem Gemisch der cyclischen Ketoenole der allgemeinen Formeln (XI), (XII) und (XIII). Deshalb ist es für den Erhalt eines sauberen Produktes der allgemeinen Formel (XI) unter technischen Bedingungen (beispielsweise keine Reinigung der Zielverbindung durch Chromatographie möglich) unerlässlich, in einer zusätzlichen Stufe dieses Gemisch der Verbindungen der allgemeinen Formeln (XI), (XII) und (XIII) mit einem Diol der allgemeinen Formel (XIV) in Gegenwart eines sauren Katalysators zu der einheitlichen Verbindung der allgemeinen Formel (XI) umzusetzen. Dieser zusätzliche Schritt ist zeit- und kostenintensiv und unökonomisch.

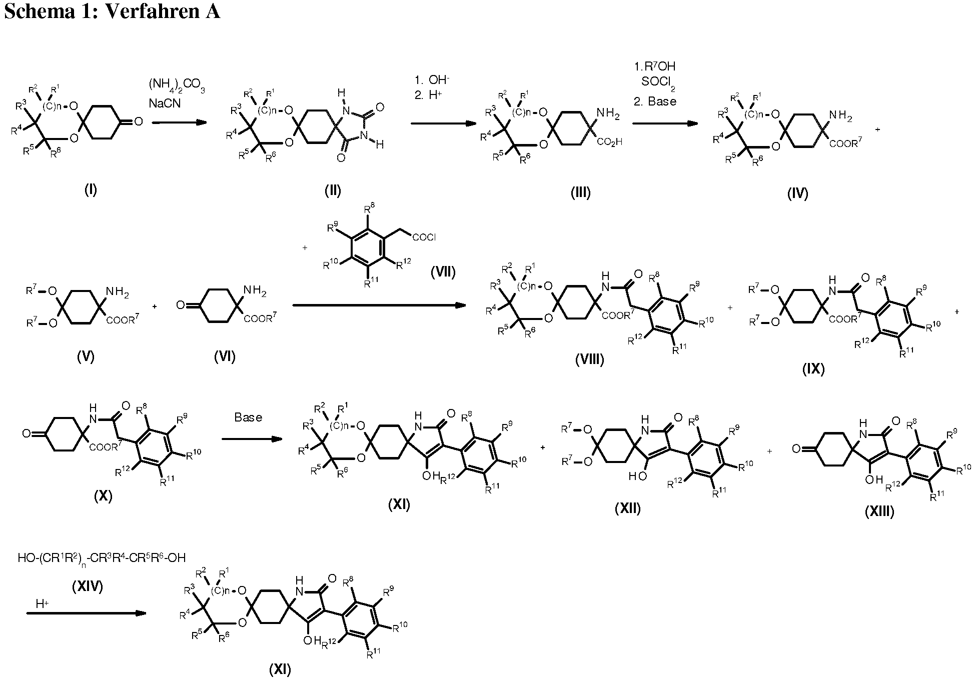

Es ist auch bereits bekannt geworden, cis-alkoxy-substituierte spirocyclische Phenylacetylaminosäureester und cis-alkoxy-substituierte spirocyclische 1-H-Pyrrolidin-2,4-dion-Derivate herzustellen, indem man beispielsweise das Natriumsalz der cis-alkoxy-substituierten spirocyclischen Phenylacetylaminosäure zuerst mit einem Chlorameisensäurester und anschließend mit einem Alkohol bzw. einem Alkoholat umsetzt (WO 2013/144101)*.* Dieses Verfahren ist jedoch sehr eng beschränkt nur für die cis-alkoxy-substituierten Verbindungen beschrieben, so dass eine Anwendung auf die chemisch wesentlich empfindlicheren spiroketal-substituierten Derivate nicht zu erwarten war.

Es bestand daher weiterhin Bedarf an einem einfacheren, kürzeren Verfahren zur Herstellung von spiroketal-substituierten Phenylacetylaminosäureestern der allgemeinen Formel (VIII) und spiroketal-substituierten cyclischen Ketoenolen der allgemeinen Formel (XI).

Es wurde nun gefunden, dass sich die Synthese von spiroketal-substituierten Phenylacetylaminosäureestern der allgemeinen Formel (VIII) und von spiroketal-substituierten cyclischen Ketoenolen der allgemeinen Formel (XI) überraschend vereinfachen lässt und dass man ein Gemisch aus Verbindungen der Formeln (VIII) und (VIII') 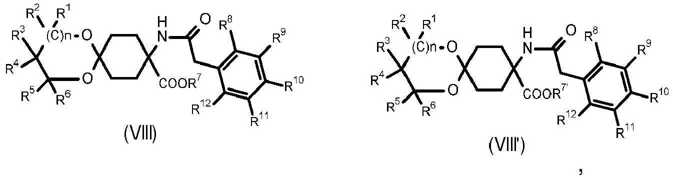 in welchen
- R¹ bis R⁶: unabhängig voneinander für Wasserstoff, Methyl, Ethyl oder Phenyl stehen,
- R⁸ bis R¹²: unabhängig voneinander für Wasserstoff, Methyl, Ethyl, Fluoralkyl mit ein oder 2 C-Atomen und ein bis fünf Fluoratomen, Halogen, Methoxy, Ethoxy, Trifluormethoxy oder gegebenenfalls durch Methyl, Ethyl, Methoxy, Ethoxy oder Halogen substituiertes Phenyl
stehen,
- n: für 0 oder 1
steht,
- R⁷: für Alkyl steht,
- R^{7'}: für Alkyl steht,
erhält,
indem man zunächst Verbindungen der Formel (XV) 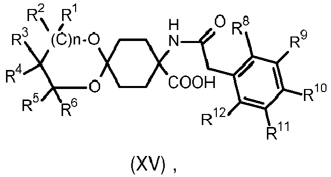 in welcher R¹ bis R⁶, R⁸ bis R¹² und n die oben genannten Bedeutungen haben,
in Gegenwart einer Base zu Verbindungen der Formel (XV') 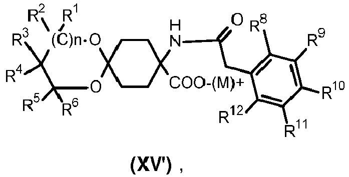
R¹ bis R⁶, R⁸ bis R¹² und n die oben genannten Bedeutungen haben und
M für ein Alkalimetallion, ein Ionenäquivalent eines Erdalkalimetalls, ein Ionenäquivalent Aluminium oder ein Ionenäquivalent eines Übergangsmetalls steht oder weiterhin
für ein Ammoniumion, bei dem gegebenenfalls ein, zwei, drei oder alle vier Wasserstoffatome durch gleiche oder verschiedene Reste aus den Gruppen C₁-C₅-Alkyl, C₁-C₅-Isoalkyl oder C₃-C₇-Cycloalkyl, die jeweils ein- oder mehrfach mit Fluor, Chlor, Brom, Cyano, Hydroxy substituiert oder durch ein- oder mehrere Sauerstoff- oder Schwefelatome unterbrochen sein können, ersetzt sein können, steht, oder weiterhin
für ein cyclisches sekundäres oder tertiäres aliphatisches oder heteroaliphatisches Ammoniumion, beispielsweise Morpholinium, Thiomorpholinium, Piperidinium, Pyrrolidinium, oder jeweils protoniertes 1,4-Diazabicyclo[2.2.2]octane (DABCO) oder 1,5-Diazabicyclo[4.3.0]undec-7-en (DBU), steht, oder weiterhin
für ein heterocyclisches Ammoniumkation, beispielsweise jeweils protoniertes Pyridin, 2-Methylpyridin, 3-Methylpyridin, 4-Methylpyridin, 2,4-Dimethylpyridin, 2,5-Di-methylpyridin, 2,6-Dimethylpyridin, 5-Ethyl-2-methylpyridin, Pyrrol, Imidazol, Chinolin, Chinoxalin, 1,2-Dimethylimidazol, 1,3-Dimethylimidazolium-methylsulfat, steht, oder weiterhin
für ein Sulfoniumion steht, oder weiterhin
für ein Magnesium-Halogen-Kation steht,
umsetzt
und mit Verbindungen der Formel (XVI) 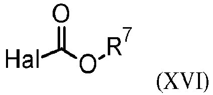
in welcher R⁷ die oben angegebenen Bedeutung hat
   und
Hal für Halogen steht,
weiter zu Verbindungen der Formel (XVII) umsetzt 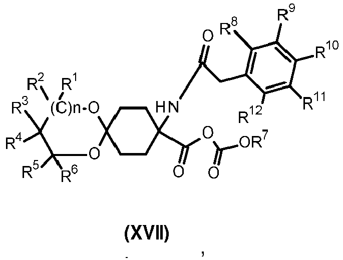 in welcher R¹ bis R¹² und n die oben genannten Bedeutungen haben,
und die weiter zu Verbindungen der Formel (XVIII) 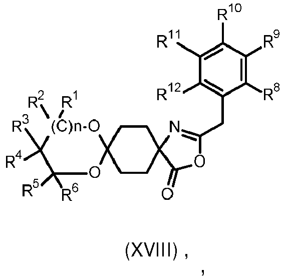 in welcher R¹ bis R¹² und n die oben genannten Bedeutungen haben, cyclisieren, die wiederum mit Verbindungen der Formel (XIX)

R^{7'}-OH (XIX),

in welcher
R^{7'} die oben angegebene Bedeutung hat,
zu einem Gemisch aus Verbindungen der Formel (VIII) und (VIII') reagieren.
R¹ bis R⁶ bedeutet R¹, R², R³, R⁴, R⁵, R⁶.
R⁸ bis R¹² bedeutet R⁸, R⁹, R¹⁰, R¹¹, R¹².

In den Formeln (I), (II), (III), (IV), (V), (VI), (VII), (VII'), (VIII), (IX), (X), (XI), (XII), (XIII), (XIV), (XV), (XV'), (XVI), (XVII), (XVIII), (XIX) und (XXI) stehen
- R¹ bis R⁶: unabhängig voneinander bevorzugt für Wasserstoff, Methyl oder Ethyl,
- R⁸ bis R¹²: unabhängig voneinander bevorzugt für Wasserstoff, Methyl, Ethyl, Fluor, Chlor, Methoxy, Ethoxy, Trifluormethoxy oder gegebenenfalls durch Methyl, Ethyl, Methoxy, Ethoxy, Fluor, Chlor oder Brom substituiertes Phenyl,
- n: bevorzugt für 0 oder 1,
- Hal: bevorzugt für Chlor, Brom, Fluor, Iod,
- R⁷: bevorzugt für C₁-C₆-Alkyl,
- R^{7'}: bevorzugt für C₁-C₆-Alkyl;
- R¹ bis R⁶: unabhängig voneinander besonders bevorzugt für Wasserstoff oder Methyl,
- R⁸ bis R¹²: unabhängig voneinander besonders bevorzugt für Wasserstoff, Methyl, Ethyl, Fluor, Chlor, Methoxy, Ethoxy oder gegebenenfalls durch Methyl, Ethyl, Methoxy, Ethoxy, Fluor oder Chlor substituiertes Phenyl,
- n: besonders bevorzugt für 0,
- Hal: besonders bevorzugt für Chlor, Brom oder Fluor,
- R⁷: besonders bevorzugt für C₁-C₄-Alkyl,
- R^{7'}: besonders bevorzugt für C₁-C₄-Alkyl;
- R¹ bis R⁶: unabhängig voneinander ganz besonders bevorzugt für Wasserstoff oder Methyl,
- R⁸ bis R¹²: unabhängig voneinander ganz besonders bevorzugt für Wasserstoff, Methyl, Ethyl, Fluor, Chlor, Methoxy, Ethoxy oder gegebenenfalls durch Methyl, Methoxy, Fluor oder Chlor substituiertes Phenyl,
- n: ganz besonders bevorzugt für 0,
- Hal: ganz besonders bevorzugt für Chlor oder Fluor,
- R⁷: ganz besonders bevorzugt für Methyl, Ethyl, Propyl, Butyl oder Isobutyl,
- R^{7'}: ganz besonders bevorzugt für Methyl, Ethyl, Propyl, Butyl oder Isobutyl;
- R¹ bis R⁶: unabhängig voneinander hervorgehoben für Wasserstoff oder Methyl,
- R⁸ bis R¹²: unabhängig voneinander hervorgehoben für Wasserstoff, Methyl, oder Chlor,
- n: hervorgehoben für 0,
- Hal: hervorgehoben für Chlor,
- R⁷: hervorgehoben für Methyl oder Ethyl,
- R^{7'}: hervorgehoben für Methyl oder Ethyl;
- R¹ bis R⁶: unabhängig voneinander insbesonders bevorzugt für Wasserstoff,
- R⁸: insbesonders bevorzugt für Methyl,
- R⁹: insbesonders bevorzugt für Wasserstoff,
- R¹⁰: insbesonders bevorzugt für Chlor,
- R¹¹: insbesonders bevorzugt für Wasserstoff,
- R¹²: insbesonders bevorzugt für Methyl,
- n: insbesonders bevorzugt für 0,
- Hal: insbesonders bevorzugt für Chlor,
- R⁷: insbesonders bevorzugt für Methyl oder Ethyl (insbesondere bevorzugt für Ethyl),
- R^{7'}: insbesonders bevorzugt für Methyl oder Ethyl (insbesondere bevorzugt für Methyl).

In der Formel (XV') steht
- M: bevorzugt für Lithium, Natrium, Kalium, Caesium, ein Ionenäquivalent Magnesium oder Calcium oder für ein Ammoniumion, bei dem gegebenenfalls ein, zwei, drei oder alle vier Wasserstoffatome durch gleiche oder verschiedene Reste aus den Gruppen C₁-C₅-Alkyl, C₁-C₅-Isoalkyl oder C₃-C₇-Cycloalkyl, die jeweils ein- oder mehrfach mit Fluor, Chlor, Brom, Cyano, Hydroxy substituiert sein können, ersetzt sein können,
- M: besonders bevorzugt für Lithium, Natrium, Kalium, Caesium, ein Ionenäquivalent Magnesium, ein Ionenäquivalent Calcium,
- M: ganz besonders bevorzugt für Lithium, Natrium, Kalium, Caesium,
- M: hervorgehoben für Natrium.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Der Verlauf des erfindungsgemäßen Verfahrens (B) wird im Reaktionsschema 2 exemplarisch wiedergegeben.

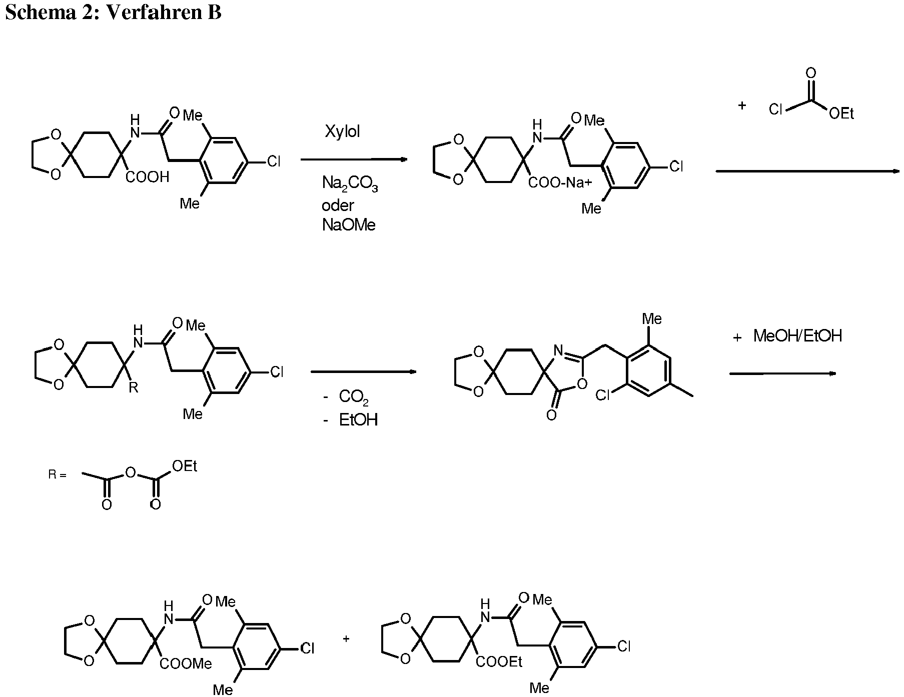

In Abhängigkeit von der Alkoholart und Alkoholmenge (Verbindungen der Formel (XIX)) wird ein Estergemisch der Formeln (VIII), (VIII') erhalten (in Schema 2: Methyl- und Ethylester).

Außerdem wurde gefunden, dass man Verbindungen der Formel (VIII') 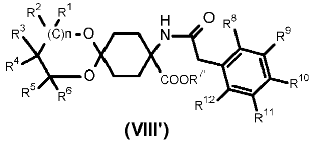 in welcher R¹ bis R⁶, R⁸ bis R¹², R^{7'} und n die oben angegebenen Bedeutungen haben,
erhält,
indem man Verbindungen der Formel (XV) 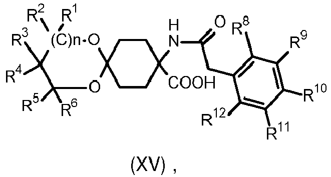 in welcher
R¹ bis R⁶, R⁸ bis R¹² und n die oben angegebenen Bedeutungen haben,
in Gegenwart einer Base
zu Verbindungen der Formel (XV') 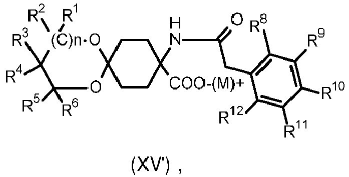 in welcher
M, R¹ bis R⁶, R⁸ bis R¹² und n die oben angegebenen Bedeutungen haben,
umsetzt
und mit Verbindungen der Formel (XVI) 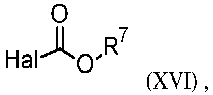 in welcher R⁷ die oben angegebenen Bedeutungen hat
und
Hal für Halogen steht,
weiter zu Verbindungen der Formel (XVII) umsetzt 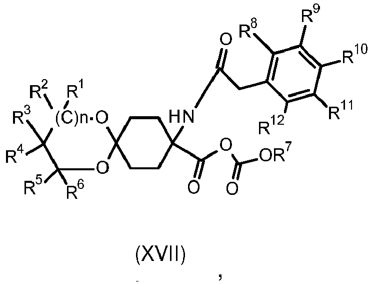 in welcher R¹ bis R⁶, R⁷, R⁸ bis R¹² und n die oben angegebenen Bedeutungen haben und die weiter zu Verbindungen der Formel (XVIII) 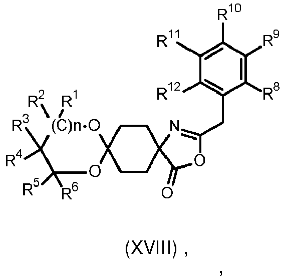 in welcher R¹ bis R⁶, R⁸ bis R¹² und n die oben angegebenen Bedeutungen haben,
cyclisieren,
die wiederum mit Verbindungen der Formel (XIX)

R^{7'}-OH (XIX),

in welcher R^{7'} die oben angegebenen Bedeutungen hat,
zu Verbindungen der Formel (VIII') 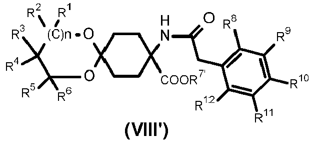 reagieren.

Es ist bekannt, dass man die Verbindungen der Formel (VIII) bzw. (VIII') in Gegenwart eines Verdünnungsmittels und in Gegenwart einer Base intramolekular zu spiroketal-substituierten cyclischen Ketoenolen der Formel (XI) 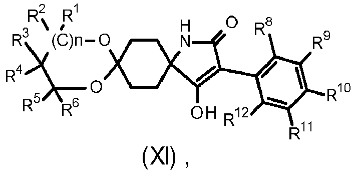 in welcher R¹ bis R⁶, R⁸ bis R¹² und n die oben angegebenen Bedeutungen haben, kondensieren kann (Dieckmann-Cyclisierung).
Jetzt wurde gefunden, dass man die Verbindungen der Formel (XI) 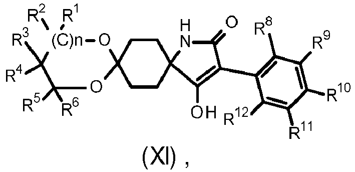 in welcher
R¹ bis R⁶, R⁸ bis R¹² und n die oben angegebenen Bedeutungen haben,
in einer vorteilhaften Eintopfreaktion erhält,
indem man Verbindungen der Formel (XV) 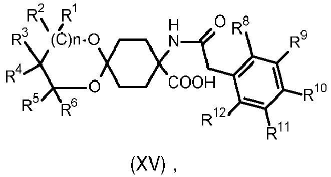 in welcher
R¹ bis R⁶, R⁸ bis R¹² und n die oben angegebenen Bedeutungen haben,
in Gegenwart einer Base
zu Verbindungen der Formel (XV') 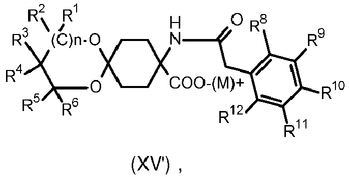 in welcher
R¹ bis R⁶, R⁸ bis R^{12,} M und n die oben angegebenen Bedeutungen haben,
umsetzt
und mit Verbindungen der Formel (XVI) 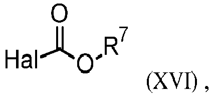 in welcher R⁷ die oben angegebenen Bedeutungen hat
und
Hal für Halogen steht,
in Gegenwart einer starken Base der Formel (XX)

ZLR^{7'} (XX),

in welcher
- Z: für ein Alkalimetall (bevorzugt für Lithium, Natrium, oder Kalium, besonders bevorzugt für Natrium oder Kalium, ganz besonders bevorzugt für Natrium) steht,
- L: für Sauerstoff oder Schwefel (bevorzugt für Sauerstoff) steht und
- R^{7'}: die oben angegebenen Bedeutungen hat,
umsetzt.

Der Verlauf des erfindungsgemäßen Eintopfverfahrens wird durch das Reaktionsschema 3 beispielhaft wiedergegeben.

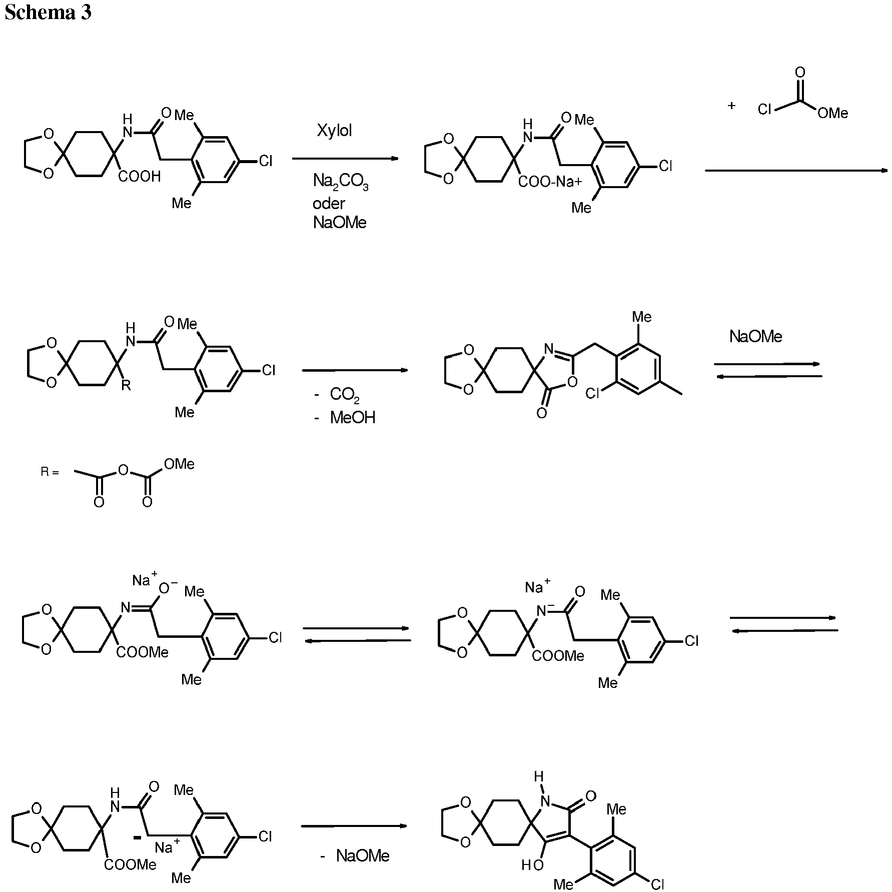

Überraschenderweise können nach dem erfindungsgemäßen Verfahren die Verbindungen der Formel (XI) auf einfachere Weise, in einem Eintopfverfahren, ohne Isolierung der Zwischenstufen, in höherer Reinheit und in besserer Ausbeute hergestellt werden.

Die Verbindungen der Formeln (XV'), (XVII) und (XVIII) sind neu.

Die Verbindungen der Formel (XV) sind bekannt (WO 06/089633) oder lassen sich nach den dort beschriebenen Verfahren herstellen.

Die Verbindungen der Formeln (XVI), (XIX), (XX) und (XX') sind kommerziell erhältlich.

Die Verbindungen der Formel (VIII) und (VIII') sind bekannt (WO 06/089633) oder lassen sich nach den dort beschriebenen Verfahren herstellen.

Die Verbindungen der Formel (XVIII) können in zwei tautomeren Formen vorliegen:

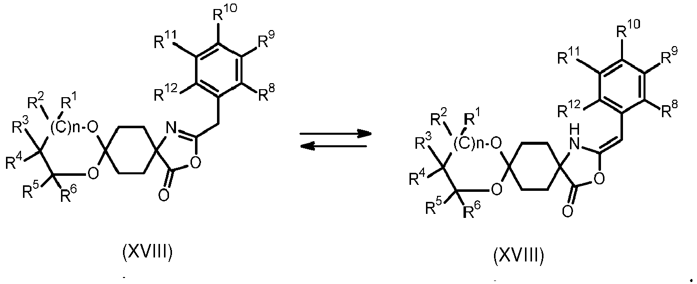

Die Verbindungen können sowohl als Gemische als auch in Form ihrer reinen Tautomeren vorliegen. Gemische lassen sich gegebenenfalls durch physikalische Methoden trennen, beispielsweise durch chromatographische Methoden.

Aus Gründen der besseren Übersichtlichkeit wird im Folgenden jeweils nur eines der möglichen Tautomeren aufgeführt. Das schließt nicht aus, dass die Verbindungen gegebenenfalls in Form der Tautomerengemische oder in der jeweils anderen tautomeren Form vorliegen können.

Die Reaktionstemperatur zur Herstellung der Verbindungen der Formel (XV') kann bei der Durchführung des erfindungsgemäßen Verfahrens variiert werden. Im Allgemeinen arbeitet man bei Temperaturen zwischen 0 °C und 110 °C, bevorzugt zwischen 10 °C und 80 °C.

Als Base können zur Herstellung der Verbindungen der Formel (XV') verwendet werden: Alkalihydroxide wie beispielsweise Lithiumhydroxid, Natriumhydroxid, Kaliumhydroxid; Erdalkalihydroxide wie beispielsweise Caclciumhydroxid; Alkalicarbonate und -hydrogencarbonate wie beispielsweise Natriumcarbonat, Natriumhydrogencarbonat, Kaliumcarbonat, Kaliumhydrogencarbonat; Alkalialkoholate wie beispielsweise Natriummethylat, Natriumethylat, Natriumtertiärbutylat, Kaliummethylat, Kaliumethylat, Kaliumtertiärbutylat. Bevorzugt sind Natriumhydroxid, Kaliumhydroxid, Natriumcarbonat, Kaliumcarbonat, Natriummethylat und Natriumethylat. Besonders bevorzugt ist Natriummethylat. Besonders bevorzugt ist auch Natriumethylat. Ebenso bevorzugt ist Natriumhydroxid.

Die Alkalialkoholate können als Feststoffe oder als Lösungen, beispielsweise als Lösung in dem entsprechenden Alkohol, also beispielsweise Natriummethylat in Methanol, Kaliumethylat in Ethanol, eingesetzt werden. Bevorzugt verwendet man die Lösungen; besonders bevorzugt eine Lösung von Natriummethylat in Methanol.

Als Lösungsmittel können Toluol, Xylol, Alkane wie n-Hexan, n-Heptan, n-Octan, Ether wie Diethy-, Diisopropyl-, Dibutylether, Anisol, Methyl-tert-butylether, Methyl-tert-amylether, Glykoldimethylether, Diglykoldimethylether, Tetrahydrofuran, 2-Methyl-tetrahydrofuran, Cyclopentyl-methyl-ether oder Dioxan, Ketone wie Aceton, Methyl-ethyl-, Methyl-isopropyl- oder Methyl- isobutyl-keton (MIBK), Kohlenwasserstoffe wie Pentan, Hexan, Heptan, Cyclohexan, Methylcyclohexan, halogenierte Kohlenwasserstoffe wie Methylenchlorid, Chloroform, Tetrachlorkohlenstoff, Chlorbenzol, o-Dichlorbenzol oder Dichlorethan verwendet werden. Bevorzugt verwendet werden Toluol oder Xylol.

Die Reaktionstemperatur zur Herstellung der Verbindungen der Formel (XV') kann bei der Durchführung des erfindungsgemäßen Verfahrens variiert werden. Im Allgemeinen arbeitet man bei Temperaturen zwischen 0 °C und 100 °C, vorzugsweise zwischen 10 °C und 80 °C.

Bei der Durchführung des erfindungsgemäßen Verfahrens setzt man die Reaktionskomponenten der Formel (XVI) im Allgemeinen in äquimolaren bis doppeltäquimolaren Mengen ein. Bevorzugt verwendet man 1,1 bis 1,5 Moläquivalente.

Das erfindungsgemäße Eintopfverfahren ist dadurch gekennzeichnet, dass man Verbindungen der Formel (XV) in Gegenwart eines Verdünnungsmittels mit Basen zu dem entsprechenden Salz der Formel (XV') umsetzt, nach gegebenenfalls azeotroper Trocknung des Reaktionsgemisches die Verbindungen der Formel (XV') mit Verbindungen der Formel (XVI) zu Zwischenverbindungen der Formel (XVII) umsetzt aus denen durch Cyclisierung die Verbindungen der Formel (XVIII) entstehen. Diese werden durch Einwirkung einer starken Base der Formel (XX) zu Verbindungen der Formel (XXI) 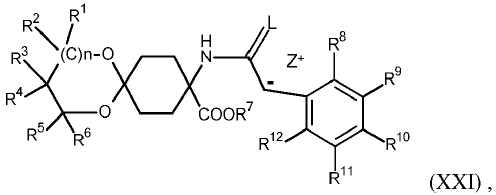 in welcher R¹ bis R⁶, R⁷, R⁸ bis R¹², L, Z und n die oben angegebenen Bedeutungen haben,
aufgespalten und zu Verbindungen der Formel (XI) cyclisiert.

Weiterhin ist es möglich, dass die Verbindungen der Formel (XVII) in Gegenwart einer starken Base direkt zu Verbindungen der Formel (XI) reagieren.

Die Verbindungen der Formel (XXI) können in folgenden tautomeren Formen auftreten:

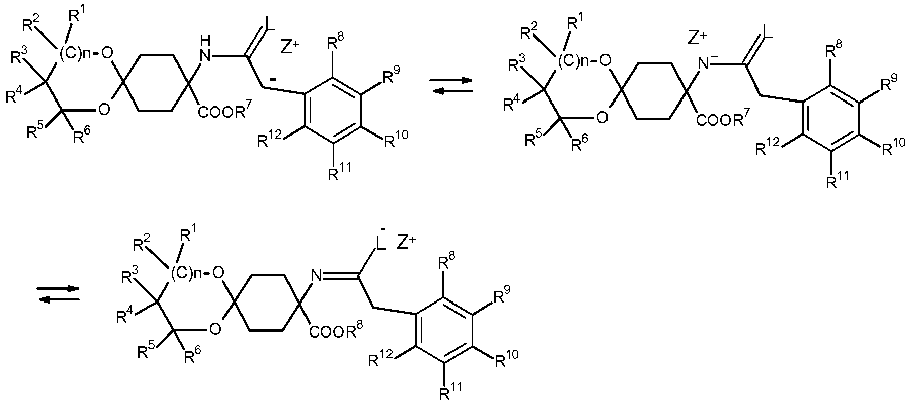

Die Verbindungen können sowohl als Gemische als auch in Form ihrer reinen Tautomeren vorliegen. Gemische lassen sich gegebenenfalls durch physikalische Methoden trennen, beispielsweise durch chromatographische Methoden.

Aus Gründen der besseren Übersichtlichkeit wird im Folgenden jeweils nur eines der möglichen Tautomeren aufgeführt. Das schließt nicht aus, dass die Verbindungen gegebenenfalls in Form der Tautomerengemische oder in der jeweils anderen tautomeren Form vorliegen können.

Die Reaktionstemperatur zur Herstellung der Verbindungen der Formel (XI) ausgehend von Verbindungen der Formel (XV) kann bei der Durchführung des erfindungsgemäßen Eintopfverfahrens variiert werden. Im Allgemeinen arbeitet man bei Temperaturen zwischen 20 °C und 110 °C, vorzugsweise zwischen 60 °C und 85 °C. Bevorzugt ist eine Temperatur von 60 °C.

Als Base können die oben genannten Verbindungen eingesetzt werden.

Als starke Base der Formel (XX) können beispielsweise Alkoholate oder Thioalkoholate sowohl als Feststoff als auch als Lösung eingesetzt werden. Zum Beispiel. NaOMe fest oder als Lösung in Methanol, NaOEt fest oder als Lösung in Ethanol, NaSMe fest oder als Lösung in Methanol, NaSEt fest oder als Lösung in Ethanol. Bevorzugt wird NaOMe als Lösung in Methanol.

Als Lösungsmittel können Toluol, Xylol, Alkane wie n-Hexan, n-Heptan, n-Octan, Ether wie Diethy-, Diisopropyl-, Dibutylether, Anisol, Methyl-tert-butylether, Methyl-tert-amylether, Glykoldimethylether, Diglykoldimethylether, Tetrahydrofuran oder Dioxan, Ketone wie Aceton, Methyl- ethyl-, Methyl-isopropyl- oder Methyl- isobutyl-keton (MIBK), Kohlenwasserstoffe wie Pentan, Hexan, Heptan, Cyclohexan, Methylcyclohexan, Benzol, halogenierte Kohlenwasserstoffe wie Methylenchlorid, Chloroform, Tetrachlorkohlenstoff, Chlorbenzol, o-Dichlorbenzol, Dichlorethan verwendet werden. Bevorzugt verwendet werden Toluol oder Xylol. Besonders bevorzugt wird Xylol verwendet.

Bei der Durchführung des erfindungsgemäßen Eintopfverfahrens setzt man die Reaktionskomponenten der Formel (XVI) im Allgemeinen in äquimolaren bis etwa doppeltäquimolaren Mengen ein.

### Herstellungsbeispiele

### Beispiel 1

### 11-(4-Chlor-2,6-dimethylphenyl)-12-hydroxy-1,4-dioxa-9-azadispiro[4.2.4.2]tetradec-11-en-10-on, Eintopfverfahren

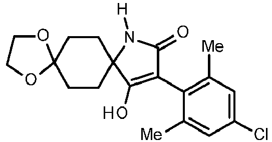

Zu einer Suspension von 4,73 g [12,37 mmol] 8-{[(4-Chlor-2,6-dimethylphenyl)acetyl]amino}-1,4-dioxaspiro[4.5]decan-8-carbonsäure in 25 ml Toluol gibt man bei Raumtemperatur 2,674 g [14,85 mmol] einer 30%igen Lösung von NaOMe in Methanol. Man rührt 30 Minuten bei Raumtemperatur, wobei eine gelbliche Lösung entsteht. Methanol und Toluol werden im Vakuum fast vollständig abdestilliert. Man gibt 25 ml frisches Toluol hinzu und tropft zu der Lösung 1,40 g [14,85 mmol] Chlorameisensäuremethylester. Nach 2 Stunden Rühren bei Raumtemperatur gibt man 20 ml trockenes N,N-Dimethylacetamid hinzu und destilliert anschließend Toluol im Vakuum ab. Man versetzt den Rückstand mit 5,57 g [39,93 mmol] einer 30%igen Lösung von NaOMe in Methanol und erhitzt für 4,5 Stunden auf 110°C. Anschließend wird restliches Methanol abdestilliert und der Rückstand in eine Mischung aus 125 ml Eiswasser und 25 ml Essigsäure eingerührt. Man saugt den ausgefallenen Feststoff ab, wäscht ihn zweimal mit je 5 ml Wasser und trocknet bei 50°C im Vakuum. Man erhält 4,60 g beigen Feststoff, der laut HPLC-Analyse gegen einen Referenzstandard eine Reinheit von 90,2%(w/w) aufweist, womit sich eine Ausbeute von 92,2% der Theorie errechnet.
¹H-NMR (600 MHz, d₆-DMSO): δ = 1.4-1.42 (m; 2H), 1,68-1,71 (m; 2H), 1,85-1,91 (m; 2H), 2,07 (s; 6H), 2,1-2.14 (m; 2H), 3,88 (s; 4H), 7,11 (s; 2H), 8,18 (s, br; 1H), 10,8 (s; 1H) ppm.

### Beispiel 2

### 11-(4-Chlor-2,6-dimethylphenyl)-12-hydroxy-1,4-dioxa-9-azadispiro[4.2.4.2]tetradec-11-en-10-on, Eintopfverfahren

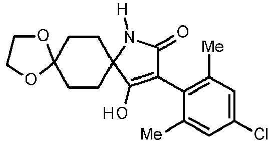

Zu einer Suspension von 14,69 g [38,48 mmol] 8-{[(4-Chlor-2,6-dimethylphenyl)acetyl]amino}-1,4-di-oxaspiro[4.5]decan-8-carbonsäure in 90 ml Toluol gibt man bei Raumtemperatur 8,32 g [46,18 mmol] einer 30%igen Lösung von NaOMe in Methanol. Man rührt 30 Minuten bei Raumtemperatur, wobei eine gelbliche Lösung entsteht. Methanol und Toluol werden im Vakuum fast vollständig abdestilliert. Man gibt 90 ml frisches Toluol hinzu und tropft zu der Lösung 4,36 g [46,18 mmol] Chlorameisensäuremethylester. Nach 1 Stunde Rühren bei Raumtemperatur gibt man 72 ml trockenes N,N-Dimethylacetamid hinzu und destilliert anschließend Toluol im Vakuum ab. Man versetzt den Rückstand mit 17,33 g [96,21 mmol] einer 30%igen Lösung von NaOMe in Methanol und erhitzt für 3 Stunden auf 110°C. Anschließend wird restliches Methanol abdestilliert und der Rückstand in eine Mischung aus 350 ml Eiswasser und 90 ml Essigsäure eingerührt. Man saugt den ausgefallenen Feststoff ab, wäscht ihn zweimal mit je 10 ml Wasser und trocknet bei 50°C im Vakuum. Man erhält 14,1 g beigen Feststoff, der laut HPLC-Analyse gegen einen Referenzstandard eine Reinheit von 94,9*%*(w/w) aufweist, womit sich eine Ausbeute von 95,5% der Theorie errechnet.

### Beispiel 3

### 11-(4-Chlor-2,6-dimethylphenyl)-12-hydroxy-1,4-dioxa-9-azadispiro[4.2.4.2]tetradec-11-en-10-on, Eintopfverfahren

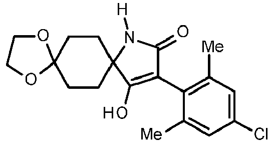

Zu einer Suspension von 1,91 g [5 mmol] 8-{[(4-Chlor-2,6-dimethylphenyl)acetyl]amino}-1,4-dioxaspiro[4.5]decan-8-carbonsäure in 10 ml N,N-Dimethylacetamid gibt man bei Raumtemperatur 1,08 g [6 mmol] einer 30%igen Lösung von NaOMe in Methanol. Man rührt 30 Minuten bei Raumtemperatur, wobei eine gelbliche Lösung entsteht. Das Methanol wird im Vakuum vollständig abdestilliert. Anschließend tropft man 0,567 g [6 mmol] Chlorameisensäuremethylester zu. Nach 2 Stunden Rühren bei Raumtemperatur gibt man weitere 0,095 g [1 mmol] Chlorameisensäuremethylester zu und rührt für 1 weitere Stunde bei Raumtemperatur. Man versetzt das Reaktionsgemisch dann mit 2,25 g [12,5 mmol] einer 30%igen Lösung von NaOMe in Methanol und erhitzt für 16 Stunden auf 110°C. Anschließend wird das Reaktionsgemisch in eine Mischung aus 50 ml Eiswasser und 15 ml Essigsäure eingerührt. Man saugt den ausgefallenen Feststoff ab, wäscht ihn zweimal mit je 5 ml Wasser und trocknet bei 50°C im Vakuum. Man erhält 1,7 g beigen Feststoff, der laut HPLC-Analyse gegen einen Referenzstandard eine Reinheit von 92,2%(w/w) aufweist, womit sich eine Ausbeute von 86,1% der Theorie errechnet.

### Beispiel 4

### 2-(4-Chlor-2,6-dimethylbenzyl)-3,9,12-trioxa-1-azadispiro[4.2.4.2]tetradec-1-en-4-on

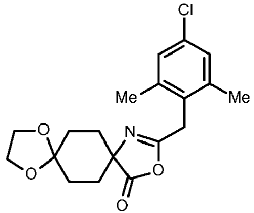

Man legt 46,89 g [122,8 mmol] 8-{[(4-Chlor-2,6-dimethylphenyl)acetyl]amino}-1,4-dioxa-spiro[4.5]decan-8-carbonsäure in 250 ml Toluol als Suspension vor. Bei Raumtemperatur tropft man 26,54 g [147,4 mmol] einer 30%igen Lösung von NaOMe in Methanol zu, wobei eine gelbliche Lösung entsteht. Man zieht Methanol und Toluol im leichten Vakuum fast vollständig ab und nimmt den Rückstand in 250 ml Toluol auf. Zu dieser Lösung tropft innerhalb von etwa 30 Minuten bei Raumtemperatur 13,93 g [147,4 mmol] Chlorameisensäuremethylester, wobei eine weiße Suspension entsteht. Man rührt noch eine Stunde und engt dann den Ansatz im Vakuum ein. Es resultieren 57,2 g weißer Feststoff, der nach quant. NMR 76,9% der Titelverbindung enthält, was einer Ausbeute von 98,4% der Theorie entspricht.
LC/MS (pos.): m/e = 364 (MH+, ³⁵Cl).
¹H-NMR (600 MHz, CD₃CN): δ =1,64-1,84 (m; 6H), 1,86-1,92 (m; 2H), 2,32 (s; 6H), 3,83 (s; 2H), 3,90 (s; 4H), 7,10 (s; 2H) ppm.

### Beispiel 5

### Methyl-8-{[(4-chlor-2,6-dimethylphenyl)acetyl]amino}-1,4-dioxaspiro[4.5]decan-8-carboxylat

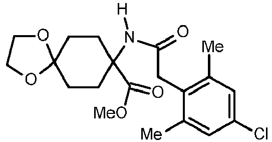

Man legt 3,82 g [10 mmol] 8-{[(4-Chlor-2,6-dimethylphenyl)acetyl]amino}-1,4-dioxaspiro[4.5]decan-8-carbonsäure in 20 ml Xylol als Suspension vor. Bei Raumtemperatur tropft man 1,8 g [10 mmol] einer 30%igen Lösung von NaOMe in Methanol zu, wobei eine gelbliche Lösung entsteht. Man zieht das Methanol im leichten Vakuum ab und tropft innerhalb von etwa 4 Stunden bei 60°C 2,08 g [22 mmol] Chlorameisensäuremethylester zu. Nach Einengen im Vakuum resultieren 4,4 g gelblicher Feststoff, der nach quant. NMR 64,0% der Titelverbindung enthält, was einer Ausbeute von 71% der Theorie entspricht.
LC/MS (pos.): m/e = 396 (MH+, ³⁵Cl), 791 ([2M+H]⁺, 2 x ³⁵Cl).

### Beispiel 6

### Methyl-8-{[(4-chlor-2,6-dimethylphenyl)acetyl]amino}-1,4-dioxaspiro[4.5]decan-8-carboxylat

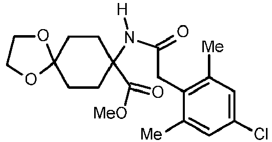

Man legt 3,82 g [10 mmol] 8-{[(4-Chlor-2,6-dimethylphenyl)acetyl]amino}-1,4-dioxaspiro[4.5]decan-8-carbonsäure in 20 ml Xylol als Suspension vor. Bei Raumtemperatur gibt man 0,89 g [10 mmol] einer 45%igen Natronlauge zu und rührt 3,5 Stunden bei Raumtemperatur. Man entfernt das Wasser durch azeotrope Destillation im leichten Vakuum ab, tropft innerhalb von 30 Minuten bei 60°C 1,134 g [12 mmol] Chlorameisensäuremethylester zu und rührt noch weitere 30 Minuten bei 60°C. Nach Einengen der so erhaltenen Lösung im Vakuum resultieren 4,2 g gelblicher Feststoff, der nach quant. NMR 69,5% der Titelverbindung enthält, was einer Ausbeute von 73,7% der Theorie entspricht.

### Beispiel 7

### Ethyl-8-{[(4-chlor-2,6-dimethylphenyl)acetyl]amino}-1,4-dioxaspiro[4.5]decan-8-carboxylat

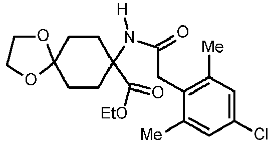

Man legt 1,0 g [2,62 mmol] 8-{[(4-Chlor-2,6-dimethylphenyl)acetyl]amino}-1,4-dioxaspiro[4.5]decan-8-carbonsäure in 5 ml Toluol als Suspension vor. Bei Raumtemperatur tropft man 1,02 g [3,14 mmol] einer 21%igen Lösung von NaOEt in Ethanol zu, wobei eine gelbliche Lösung entsteht. Man zieht Ethanol und Toluol im leichten Vakuum fast vollständig ab, löst den Rückstand in 5 ml frischem Toluol und tropft innerhalb von 30 Minuten bei Raumtemperatur 0,341 g [3,14 mmol] Chlorameisensäureethylester zu. Man rührt über Nacht, gibt 5 ml Ethanol hinzu und erhitzt 1 Stunde am Rückfluss. Nach Einengen im Vakuum resultieren 1,3 g fast weißer Feststoff, der nach quant. NMR % der Titelverbindung enthält, was einer Ausbeute von % der Theorie entspricht.
LC/MS (pos.): m/e = 410 (MH+, ³⁵Cl), 819 ([2M+H]⁺, 2 x ³⁵Cl).

## Patentansprüche

1. Verfahren zur Herstellung von Verbindungen der Formel (VIII) und (VIII') 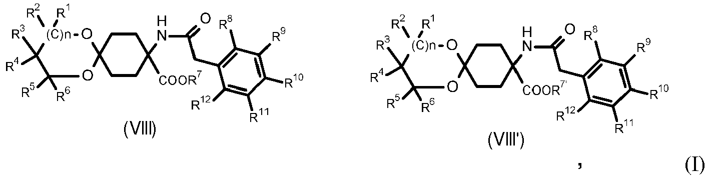 in welchen
R¹ bis R⁶ unabhängig voneinander für Wasserstoff, Methyl, Ethyl oder Phenyl stehen,
R⁸ bis R¹² unabhängig voneinander für Wasserstoff, Methyl, Ethyl, Fluoralkyl mit ein oder 2 C-Atomen und ein bis fünf Fluoratomen, Halogen, Methoxy, Ethoxy, Trifluormethoxy oder gegebenenfalls durch Methyl, Ethyl, Methoxy, Ethoxy oder Halogen substituiertes Phenyl stehen,
n für 0 oder 1 steht,
R⁷ für Alkyl steht,
R^{7'} für Alkyl steht,
**dadurch gekennzeichnet, dass** man zunächst
Verbindungen der Formel (XV) 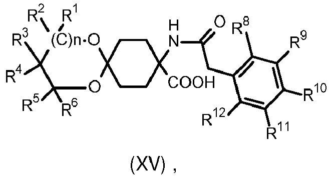 in welcher R¹ bis R⁶, R⁸ bis R¹² und n die oben genannten Bedeutungen haben,
in Gegenwart einer Base zu Verbindungen der Formel (XV') 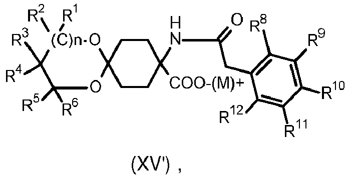 in welcher
R¹ bis R⁶, R⁸ bis R¹² und n die oben genannten Bedeutungen haben und
M für ein Alkalimetallion, ein Ionenäquivalent eines Erdalkalimetalls, ein Ionenäquivalent Aluminium oder ein Ionenäquivalent eines Übergangsmetalls steht oder weiterhin
für ein Ammoniumion, bei dem gegebenenfalls ein, zwei, drei oder alle vier Wasserstoffatome durch gleiche oder verschiedene Reste aus den Gruppen C₁-C₅-Alkyl, C₁-C₅-Isoalkyl oder C₃-C₇-Cycloalkyl, die jeweils ein- oder mehrfach mit Fluor, Chlor, Brom, Cyano, Hydroxy substituiert oder durch ein- oder mehrere Sauerstoff- oder Schwefelatome unterbrochen sein können, ersetzt sein können, steht, oder weiterhin
für ein cyclisches sekundäres oder tertiäres aliphatisches oder heteroaliphatisches Ammoniumion, beispielsweise Morpholinium, Thiomorpholinium, Piperidinium, Pyrrolidinium, oder jeweils protoniertes 1,4-Diazabicyclo[2.2.2]octane (DABCO) oder 1,5-Diazabicyclo[4.3.0]undec-7-en (DBU), steht, oder weiterhin
für ein heterocyclisches Ammoniumkation, beispielsweise jeweils protoniertes Pyridin, 2-Methylpyridin, 3-Methylpyridin, 4-Methylpyridin, 2,4-Dimethylpyridin, 2,5-Di-methylpyridin, 2,6-Dimethylpyridin, 5-Ethyl-2-methylpyridin, Pyrrol, Imidazol, Chinolin, Chinoxalin, 1,2-Dimethylimidazol, 1,3-Dimethylimidazolium-methylsulfat, steht, oder weiterhin
für ein Sulfoniumion steht, oder weiterhin
für ein Magnesium-Halogen-Kation steht,
umsetzt
und mit Verbindungen der Formel (XVI) 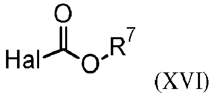 in welcher R⁷ die oben angegebenen Bedeutungen hat und Hal für Halogen steht,
weiter zu Verbindungen der Formel (XVII) 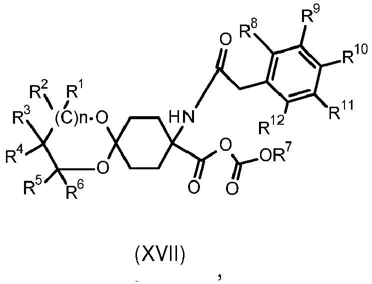 in welcher R¹ bis R⁶, R⁷, R⁸ bis R¹² und n die oben genannten Bedeutungen haben, umsetzt und
die weiter zu Verbindungen der Formel (XVIII) 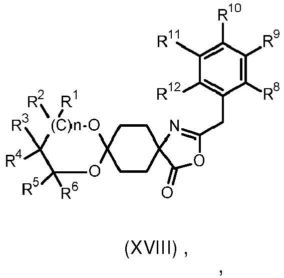 in welcher R¹ bis R⁶, R⁸ bis R¹² und n die oben genannten Bedeutungen haben, cyclisieren,
und diese wiederum
mit Verbindungen der Formel (XIX)
R^{7'}-OH (XIX)
in welcher
R^{7'} die oben angegebenen Bedeutungen hat
umsetzt.

2. Verfahren gemäß Anspruch 1, wobei
R¹ bis R⁶ unabhängig voneinander für Wasserstoff, Methyl oder Ethyl,
R⁸ bis R¹² unabhängig voneinander für Wasserstoff, Methyl, Ethyl, Fluor, Chlor, Methoxy, Ethoxy, Trifluormethoxy oder gegebenenfalls durch Methyl, Ethyl, Methoxy, Ethoxy, Fluor, Chlor oder Brom substituiertes Phenyl,
n für 0 oder 1,
Hal für Chlor, Brom, Fluor, Iod,
R⁷ für C₁-C₆-Alkyl,
R^{7'} für C₁-C₆-Alkyl
steht.

3. Verfahren gemäß Anspruch 1, wobei
R¹ bis R⁶ unabhängig voneinander für Wasserstoff oder Methyl,
R⁸ bis R¹² unabhängig voneinander für Wasserstoff, Methyl, Ethyl, Fluor, Chlor, Methoxy, Ethoxy oder gegebenenfalls durch Methyl, Ethyl, Methoxy, Ethoxy, Fluor oder Chlor substituiertes Phenyl,
n für 0,
Hal für Chlor, Brom oder Fluor,
R⁷ für C₁-C₄-Alkyl,
R^{7'} für C₁-C₄-Alkyl
steht.

4. Verfahren gemäß Anspruch 1, wobei
R¹ bis R⁶ unabhängig voneinander für Wasserstoff oder Methyl,
R⁸ bis R¹² unabhängig voneinander für Wasserstoff, Methyl, Ethyl, Fluor, Chlor, Methoxy, Ethoxy oder gegebenenfalls durch Methyl, Methoxy, Fluor oder Chlor substituiertes Phenyl,
n für 0,
Hal für Chlor oder Fluor,
R⁷ für Methyl, Ethyl, Propyl, Butyl oder Isobutyl,
R^{7'} für Methyl, Ethyl, Propyl, Butyl oder Isobutyl
steht.

5. Verfahren gemäß Anspruch 1, wobei
R¹ bis R⁶ unabhängig voneinander für Wasserstoff oder Methyl,
R⁸ bis R¹² unabhängig voneinander für Wasserstoff, Methyl oder Chlor,
n für 0,
Hal für Chlor,
R⁷ für Methyl oder Ethyl,
R^{7'} für Methyl oder Ethyl
steht.

6. Verfahren gemäß Anspruch 1, wobei
R¹ bis R⁶ für Wasserstoff,
R⁸ und R¹² für Methyl,
R¹⁰ für Chlor,
R⁹ und R¹¹ für Wasserstoff,
n für 0,
Hal für Chlor,
R⁷ für Methyl oder Ethyl,
R^{7'} für Methyl oder Ethyl
steht.

7. Verfahren gemäß der Ansprüche 1, 2, 3, 4, 5 oder 6, wobei M für Lithium, Natrium, Kalium, Caesium, Magnesium, Calcium oder für ein Ammoniumion steht, bei dem gegebenenfalls ein, zwei, drei oder alle vier Wasserstoffatome durch gleiche oder verschiedene Reste aus den Gruppen C₁-C₅-Alkyl, C₁-C₅-Isoalkyl oder C₃-C₇-Cycloalkyl, die jeweils ein- oder mehrfach mit Fluor, Chlor, Brom, Cyano, Hydroxy substituiert sein können, ersetzt sein können.

8. Verfahren gemäß der Ansprüche 1, 2, 3, 4, 5 oder 6, wobei
M für Lithium, Natrium, Kalium, Caesium, Magnesium oder Calcium
steht.

9. Verfahren gemäß der Ansprüche 1, 2, 3, 4, 5 oder 6, wobei
M für Lithium, Natrium, Kalium oder Caesium
steht.

10. Verfahren gemäß der Ansprüche 1, 2, 3, 4, 5 oder 6, wobei
M für Natrium
steht.

11. Verfahren gemäß Anspruch 1, wobei als Base Natrimmethylat eingesetzt wird.

12. Verfahren gemäß Anspruch 1, wobei als Base Natriumethylat eingesetzt wird.

13. Verfahren gemäß Anspruch 1, wobei als Base Natriumhydroxid eingesetzt wird.

14. Verbindungen der Formel (XV') 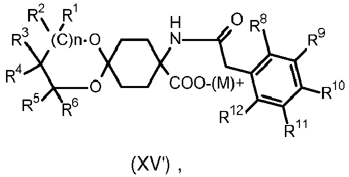 in welcher
R¹ bis R⁶, R⁸ bis R¹², M und n die Bedeutungen gemäß Anspruch 1 haben.

15. Verbindungen der Formel (XVII) 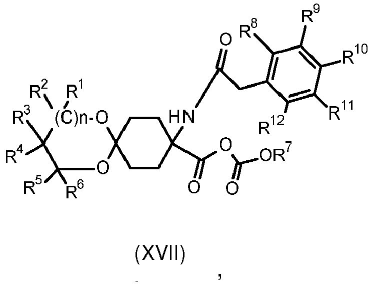 in welcher
R¹ bis R⁶, R⁷, R⁸ bis R¹² und n die Bedeutungen gemäß Anspruch 1 haben.

16. Verbindungen der Formel (XVIII) 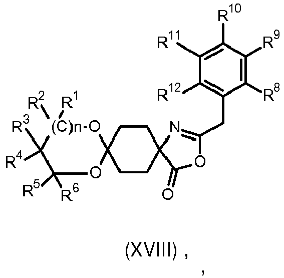 in welcher
R¹ bis R⁶, R⁸ bis R¹² und n die Bedeutungen gemäß Anspruch 1 haben.

17. Verfahren zur Herstellung von Verbindungen der Formel (VIII') 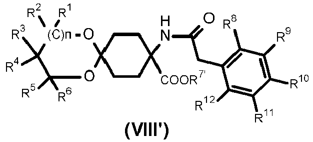 in welcher
R¹ bis R⁶, R^{7'}, R⁸ bis R¹² und n die Bedeutungen gemäß Anspruch 1 haben,
**dadurch gekennzeichnet, dass** man zunächst Verbindungen der Formel (XV) 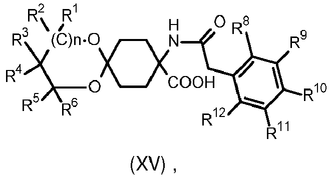 in welcher R¹ bis R⁶, R⁸ bis R¹² und n die oben genannten Bedeutungen haben, in Gegenwart einer Base zu Verbindungen der Formel (XV') 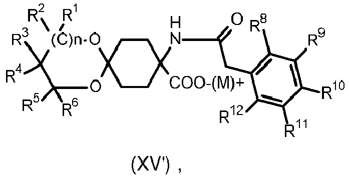 in welcher
R¹ bis R⁶, R⁸ bis R¹², M und n die oben genannten Bedeutungen haben
umsetzt
und mit Verbindungen der Formel (XVI) 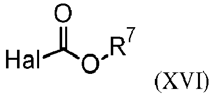 in welcher R⁷ die oben angegebenen Bedeutungen hat
und Hal für Halogen steht,
weiter zu Verbindungen der Formel (XVII) 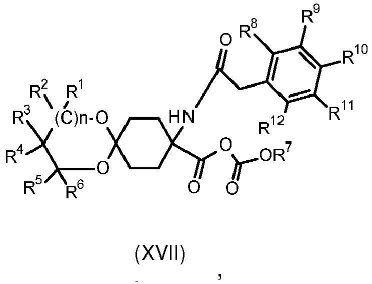 in welcher R¹ bis R⁶, R⁷, R⁸ bis R¹² und n die oben genannten Bedeutungen haben, umsetzt und
die weiter zu Verbindungen der Formel (XVIII) 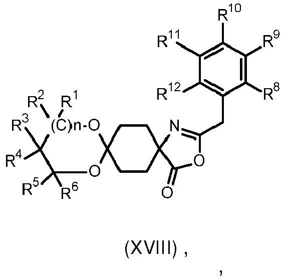 in welcher R¹ bis R⁶, R⁸ bis R¹² und n die oben genannten Bedeutungen haben, cyclisieren,
und diese wiederum
mit Verbindungen der Formel (XIX)
R^{7'}-OH (XIX)
in welcher
R^{7'} die oben angegebenen Bedeutungen hat umsetzt.

18. Verfahren zur Herstellung von Verbindungen der Formel (XI) 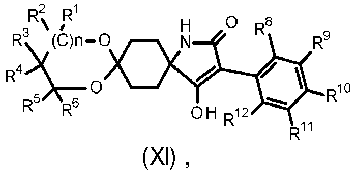 in welcher
R¹ bis R⁶, R⁸ bis R¹² und n die oben genannten Bedeutungen haben,
**dadurch gekennzeichnet, dass** man zunächst Verbindungen der Formel (XV) 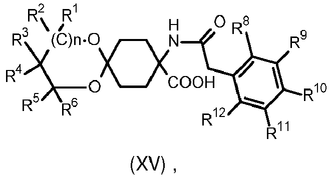 in welcher R¹ bis R⁶, R⁸ bis R¹² und n die oben genannten Bedeutungen haben, in Gegenwart einer Base zu Verbindungen der Formel (XV') 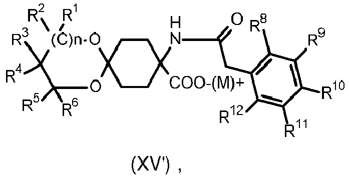 in welcher
R¹ bis R⁶, R⁸ bis R¹², M und n die oben genannten Bedeutungen haben umsetzt
und mit Verbindungen der Formel (XVI) 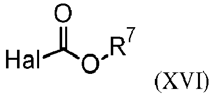 in welcher R⁷ die oben angegebenen Bedeutungen hat und Hal für Halogen steht,
in Gegenwart einer starken Base der Formel (XX)
ZLR^{7'} (XX)
in welcher
Z für ein Alkalimetall steht,
L für Sauerstoff oder Schwefel steht,
und
R^{7'} die Bedeutungen gemäß Anspruch 1 hat,
umsetzt

19. Verfahren gemäß Anspruch 18, wobei als Base und starke Base Natriummethylat eingesetzt wird

20. Verbindungen der Formel (XXI) 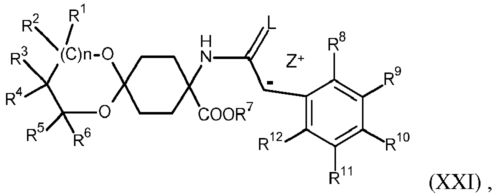 in welcher R¹ bis R⁶, R⁷, R⁸ bis R¹², L, Z und n die oben angegebenen Bedeutungen haben,
